(19) Europäisches Patentamt
European Patent Office
Office européen des brevets

(11) EP 0 687 256 B1

(12) # EUROPÄISCHE PATENTSCHRIFT

(45) Veröffentlichungstag und Bekanntmachung des Hinweises auf die Patenterteilung:
**03.09.1997 Patentblatt 1997/36**

(21) Anmeldenummer: **94908337.2**

(22) Anmeldetag: **18.02.1994**

(51) Int Cl.[6]: **C07D 271/08**, C07D 413/12, A61K 31/41

(86) Internationale Anmeldenummer:
**PCT/EP94/00459**

(87) Internationale Veröffentlichungsnummer:
**WO 94/20478 (15.09.1994 Gazette 1994/21)**

(54) **HYDROXYMETHYLFURAZANCARBONSÄUREDERIVATE UND IHRE VERWENDUNG IN DER BEHANDLUNG VON KARDIOVASKULÄREN ERKRANKUNGEN**

HYDROXYMETHYL FURAZANE CARBOXYLIC ACID DERIVATIVES AND THEIR USE IN THE TREATMENT OF CARDIO-VASCULAR CONDITIONS

DERIVES DE L'ACIDE HYDROXYMETHYLFURAZANNE-CARBOXYLIQUE ET LEUR UTILISATION DANS LE TRAITEMENT DE MALADIES CARDIO-VASCULAIRES

(84) Benannte Vertragsstaaten:
**AT BE CH DE DK ES FR GB GR IT LI NL PT SE**

(30) Priorität: **06.03.1993 DE 4307105**

(43) Veröffentlichungstag der Anmeldung:
**20.12.1995 Patentblatt 1995/51**

(73) Patentinhaber: **HOECHST AKTIENGESELLSCHAFT**
**60386 Frankfurt am Main (DE)**

(72) Erfinder:
- **SCHÖNAFINGER, Karl**
  **D-63755 Alzenau (DE)**
- **BOHN, Helmut**
  **D-61137 Schöneck (DE)**

(56) Entgegenhaltungen:
**EP-A- 0 038 438**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 687 256 B1

Printed by Jouve, 75001 PARIS (FR)

## Beschreibung

Die vorliegende Erfindung betrifft Hydroxymethylfurazancarbonsäurederivate, Verfahren zu ihrer Herstellung und ihre Verwendung.

Verschiedene 2-Oxy- und 5-Ozyfurazancarbonsäurederivate, die als Substituenten am Furazanring eine Methylgruppe tragen, sind bereits bekannt und z.B. in der EP-B 38438 oder EP-B 54873 beschrieben. 2-Oxy- und 5-Oxyfurazancarbonsäurederivate, die eine Hydroxymethylgruppe als Substituenten tragen, sind bisher allerdings nicht beschrieben.

Die vorliegende Erfindung betrifft Hydroxymethylfurazancarbonsäurederivate der allgemeinen Formel I,

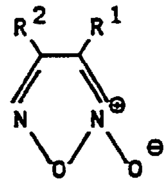

(I)

worin einer der Reste $R^1$ und $R^2$ für Hydroxymethyl und der andere für

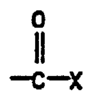

steht, wobei

X für $NR^3R^4$ oder OH oder $OR^7$ steht;
$R^3$ und $R^4$ unabhängig voneinander Wasserstoff, $(C_1-C_{20})$-Alkyl, 1-Phenyl-$(C_2-C_4)$-alkyl, 2-Phenyl-$(C_3-C_4)$-alkyl, $(C_3-C_6)$-Alkenyl, $(C_3-C_7)$-Cycloalkyl, $-(CH_2)_n-NR^5R^6$, $-(CH_2)_n-OR^5$, $-(CH_2)_m-COOR^5$, $-CH(Alk)-COOR^5$, $-(CH_2)_m-CONR^5R^6$, $-CH(Alk)-CONR^5R^6$,

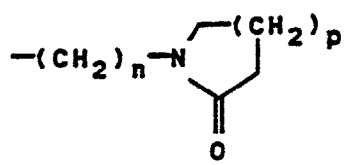

,

$-(CH_2)_n-NR^5$ (COAlk), $-(CH_2)_n$-Ar oder $-(CH_2)_n$-Het bedeuten oder $R^3$ und $R^4$ zusammen mit dem diese bindenden Stickstoffatom einen Heterocyclus bilden, der auch durch $(C_1-C_6)$-Alkyl, $(C_3-C_7)$-Cycloalkyl, $(C_1-C_4)$-Alkoxy, Amino, $(C_1-C_4)$-Alkylamino, Di($(C_1-C_4)$-alkyl)amino, Hydroxy, Acetoxy, Benzyl, Phenethyl oder Ar ein- oder mehrfach substituiert sein kann;
$R^5$ und $R^6$ unabhängig voneinander Wasserstoff, $(C_1-C_6)$-Alkyl, $(C_3-C_6)$-Alkenyl, $(C_3-C_7)$-Cycloalkyl, Benzyl, Phenethyl oder Ar bedeuten;
$R^7$ $(C_1-C_6)$-Alkyl, $(C_3-C_7)$-Cycloalkyl, Benzyl, Phenyl oder durch $(C_1-C_4)$-Alkyl, Fluor, Chlor oder Nitro ein- oder mehrfach substituiertes Phenyl bedeutet;
Alk $(C_1-C_6)$-Alkyl bedeutet;
Ar einen Arylrest mit 6 bis 12 C-Atomen, der auch durch $(C_1-C_4)$-Alkyl, $(C_1-C_4)$-Alkoxy, Amino, $(C_1-C_4)$-Alkylamino, Di($(C_1-C_4)$-alkyl)amino, $(C_1-C_6)$-Alknoylamino, Sulfamoyl, Fluor, Chlor, Brom, Hydroxy, Acetoxy, Nitro, Trifluormethyl oder Cyano ein- oder mehrfach substituiert sein kann, bedeutet;
Het einen heterocyclischen Rest mit 1 bis 3 Heteroatomen, der auch durch $(C_1-C_4)$-Alkyl, $(C_1-C_4)$-Alkoxy, Amino, $(C_1-C_4)$-Alkylamino, Di($(C_1-C_4)$-alkyl)amino, $(C_1-C_6)$-Alkanoylamino, Fluor, Chlor, Brom, Hydroxy, Acetoxy, Nitro, Cyano oder Ar ein- oder mehrfach substituiert sein kann, bedeutet;
n für 0, 1, 2, 3 oder 4,
m für 1, 2, 3 oder 4,
p für 1, 2 oder 3 steht;

sowie deren pharmakologisch annehmbare Salze.

Die vorliegende Erfindung umfaßt also sowohl Verbindungen der allgemeinen Formel Ia als auch Verbindungen der allgemeinen Formel Ib,

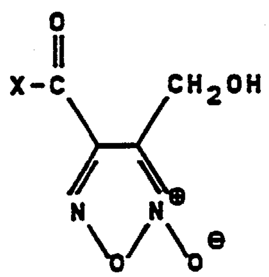

(Ia)

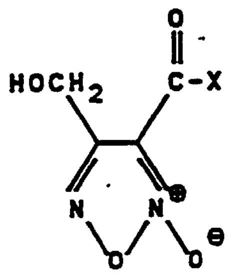

(Ib)

wobei X für $NR^3R^4$, OH oder $OR^7$ steht und $R^3$, $R^4$ und $R^7$ die angegebenen Bedeutungen haben; sie umfaßt auch Mischungen der Verbindungen der allgemeinen Formeln Ia und Ib in beliebigen Mengenverhältnissen.

Alkylgruppen können geradkettig oder verzweigt sein. Dies gilt auch, wenn sie substituiert sind oder z.B. in Alkoxygruppen oder Alkylaminogruppen oder als Substituenten an anderen Resten vorliegen. Beispiele für die für $R^3$, $R^4$, $R^5$, $R^6$, $R^7$ oder Alk stehenden Alkylgruppen sind Methyl, Ethyl, n-Propyl, i-Propyl, n-Butyl, i-Butyl, sek.-Butyl, tert.-Butyl, Pentyl, Hexyl oder 1-Methylpentyl und für die für $R^3$ oder $R^4$ stehenden Alkylgruppen auch Octyl, Decyl, Dodecyl, Hexadecyl, Octadecyl oder Eicosyl.

Auch die für $R^3$, $R^4$, $R^5$ oder $R^6$ stehenden ($C_3$-$C_6$)-Alkenylgruppen können geradkettig oder verzweigt sein. Beispiele sind Allyl, 2-Butenyl, 3-Butenyl, 2-Methyl-2-butenyl, 3-Methyl-2-butenyl, 2,3-Dimethyl-2-butenyl, 2-Hexenyl, 5-Hexenyl.

Der für $R^3$, $R^4$, $R^5$, $R^6$ oder $R^7$ oder als Substituent am aus $R^3$ und $R^4$ und dem diese bindenden Stickstoffatom gebildeten Heterocyclus stehende ($C_3$-$C_7$)-Cycloalkylrest ist beispielsweise ein Cyclopropyl-, ein Cyclobutyl-, ein Cyclopentyl-, ein Cyclohexyl- oder ein Cycloheptylrest. Bevorzugte cycloalkylreste sind der Cyclopentyl- und der Cyclohexylrest.

Der in der $(CH_2)_n$-Ar-Gruppe, in $R^5$ und $R^6$ und als Substitutent am heterocyclischen Ring Het und am aus $R^3$ und $R^4$ und dem diese bindenden Stickstoffatom gebildeten Heterocyclus enthaltene Arylrest mit 6 bis 12 C-Atomen kann beispielsweise ein unsubstituierter oder substituierter Phenyl-, 1-Naphthyl-, 2-Naphthyl-, 3-Biphenylyl- oder 4-Biphenylylrest sein. Bevorzugt ist er ein unsubstituierter oder substituierter Phenylrest.

$-(CH_2)_n$-Ar steht bevorzugt für Phenyl, Benzyl und Phenethyl, das unsubstituiert oder im Phenylring ein- oder zweifach substituiert sein kann.

Der in der $-(CH_2)_n$-Het-Gruppe enthaltene heterocyclische Rest mit 1 bis 3 Heteroatomen kann aromatisch, teilweise ungesättigt und gesättigt sein und kann anelliert sein. Bevorzugte Ringgrößen des Heterocyclus sind 5-Ringe, 6-Ringe und 7-Ringe. Bevorzugte Heteroatome sind Stickstoff, Sauerstoff und Schwefel. Beispiele für Heterocyclen, von denen sich der Rest Het ableitet, sind Azetidin, Pyrrolidin, Pyrrol, Indol, Pyrazol, Imidazolidin, Imidazolin, Imidazol, 1,2,3-Triazol, 1,2,4-Triazol, Tetrahydrofuran, Furan, 1,3-Dioxolan, Tetrahydrothiophen, Thiophen, Benzothiophen, 1,3-Dithiolan, 1,3-Oxazolin, 1,3-Oxazol, 1,3,4-Oxadiazol, Furazan, 1,3-Thiazolidin, 1,3-Thiazol, Piperidin, 1,2,5,6-Tetrahydropyridin, 1,4-Dihydropyridin, Pyridin, Chinolin, Isochinolin, Pyridazin, Pyrimidin, Piperazin, 1,2,3-Triazin, 1,3,5-Triazin, Perhydropyran, 1,3-Dioxan, 1,4-Dioxan, 1,3-Dithian, Dihydro-1,3-oxazin, Morpholin, Perhydro-1,4-thiazin, Perhydroazepin. Het kann aber auch beispielsweise ein Tetrazolylrest sein. Stickstoffheterocyclen können über ein Stickstoffatom oder ein Kohlenstoffatom gebunden sein. Bevorzugte heterocyclische Reste Het sind der 1-pyrralylrest, der 1- und der 2-Imidazolylrest, der 2-, 3-und der 4-Pyridylrest, der 4-Piperidinylrest und über ein Stickstoffatom gebundene Reste gesättigter Heterocyclen, beispielsweise der Pyrrolidino-, der Piperidino-, der Piperazino-, der Morpholino- und der Perhydro-1,4-thiazin-4-yl-Rest.

Die Arylreste Ar und die heterocyclischen Reste Het können auch ein- oder mehrfach substituiert sein. Beispiele für mögliche ($C_1$-$C_4$)-Alkylsubstituenten sind Methyl, Ethyl, n-Propyl, i-Propyl, i-Butyl oder tert.-Butyl, für mögliche ($C_1$-$C_4$)-Alkoxysubstituenten Methoxy, Ethoxy, n-Propoxy, i-Propoxy, n-Butoxy oder i-Butoxy, für mögliche ($C_1$-$C_4$)-Alkylaminosubstituenten Methylamino, Ethylamino, 1-Propylamino, 1-Butylamino oder tert.-Butyl-amino, für mögliche Di(($C_1$-$C_4$)-Alkyl)aminosubstituenten Dimethylamino, Diethylamino, Diisopropylmino, Methylethylamino, Methyl-tert.-butylamino, für ($C_1$-$C_6$)-Alkanoylamino Formylamino, Acetylamino, Propionylamino, n-Butyrylamino, i-Butyrylamino, Pivaloylamino oder Hexanoylamino. Beispiele für substituierte Derivate des als Arylrest bevorzugten Phenylrests sind

2-, 3- oder 4-Methylphenyl, 4-tert.-Butylphenyl, 2-, 3- oder 4-Methoxyphenyl, 3-Ethoxyphenyl, 2,3-, 3,4- oder 3,5-Dimethoxyphenyl, 3,4,5-Trimethoxyphenyl, 3-Aminophenyl, 3- oder 4-Dimethylaminophenyl, 4-Acetylazinophenyl, 2-, 3- oder 4-Fluorphenyl, 2,3- oder 3,4-Difluorphenyl, 2-, 3- oder 4-Chlorphenyl, 2,3-, 3,4-, 3,5- oder 2,6-Dichlorphenyl, 4-Bromphenyl, 4-Hydroxyphenyl, 3-Hydroxy-4-methoxyphenyl, 2-, 3- oder 4-Nitrophenyl, 4-chlor-3-nitrophenyl, 3- oder 4-Trifluormethylphenyl, 2-, 3- oder 4-Cyanphenyl. Beispiele für substituierte heterocyclische Reste Het sind 2,5-Dimethyl-1-pyrrolyl, 2,5-Dimethylpyrrolidino, 2,6-Dimethylpiperidino, 4-Methylpiperazino, 4-Phenylpiperazino, 4-(2-Methoxyphenyl)-piperazino, 4-Hydroxypiperidino, 4-Aminopiperidino, 4-Acetylpiperazino.

Ein aus $R^3$ und $R^4$ und dem diese bindenden Stickstoffatom gebildeter Heterocyclus ist beispielsweise Pyrrolidin, Piperidin, Morpholin oder Piperazin, wobei von den Substituenten, die dieser Heterocyclus tragen kann, am zweiten Stickstoffatom des Piperazins bevorzugt $(C_1-C_6)$-Alkyl, $(C_3-C_7)$-Cycloalkyl, Benzyl, Phenethyl und Ar stehen.

$R^7$ ist bevorzugt ein geradkettiger oder verzweigter $(C_1-C_4)$-Alkylrest, beispielsweise Methyl, Ethyl, n-Propyl, i-Propyl, n-Butyl, i-Butyl, t-Butyl, insbesondere Methyl.

Der Rest X kann chiral oder achiral vorliegen. Im Falle eines chiralen Restes X kann die Verbindung in racemischer Form oder in Form von optischen Antipoden oder Diastereomeren vorliegen.

Bevorzugt steht X für $NR^3R^4$ und einer der Reste $R^3$ und $R^4$ bedeutet Wasserstoff und der andere hat eine der angegebenen Bedeutungen oder die Reste $R^3$ und $R^4$ bilden zusammen mit dem diese bindenden Stickstoffatom einen Heterocyclus, der auch durch $(C_1-C_6)$-Alkyl, $(C_3-C_7)$-Cycloalkyl, $(C_1-C_4)$-Alkoxy, Amino, $(C_1-C_4)$-Alkylamino, Di($(C_1-C_4)$-alkyl)amino, Hydrozy, Acetoxy, Benzyl, Phenethyl oder Ar ein- oder mehrfach substituiert sein kann. Besonders bevorzugt steht einer der Reste $R^3$ und $R^4$ für Wasserstoff, und der andere steht für Wasserstoff, $(C_1-C_6)$-Alkyl, $-(CH_2)_nNR^5R^6$, wobei n für 1, 2, 3 oder 4 steht und $R^5$ und $R^6$ für $(C_1-C_6)$-Alkyl stehen, $-(CH_2)_nOR^5$, wobei n für 2, 3 oder 4 steht und $R^5$ für Wasserstoff oder $(C_1-C_4)$-Alkyl steht, $-(CH_2)_nAr$, wobei n für 0, 1, 2, 3 oder 4 steht und Ar für unsubstituiertes oder ein- oder mehrfach substituiertes Phenyl steht, oder $-(CH_2)_nHet$, wobei n für 1, 2, 3 oder 4 steht und Het einen heterocyclischen Rest bedeutet, der als Heteroatome 1 oder 2 Stickstoffatome oder 1 Stickstoffatom und 1 Sauerstoffatom enthält und durch Methylgruppen ein- oder mehrfach substituiert sein kann, oder die Reste $R^3$ und $R^4$ bilden zusammen mit dem diese bindenden Stickstoffatom einen Heterocyclus, der auch durch Methyl ein- oder mehrfach substituiert sein kann.

Darüber hinaus ist es bevorzugt, wenn einer der Reste $R^3$ und $R^4$ für Wasserstoff steht und der andere Wasserstoff, $(C_1-C_6)$-Alkyl, $-(CH_2)_nNR^5R^6$, wobei n für 1, 2 oder 3 steht und $R^5$ und $R^6$ für $(C_1-C_6)$-Alkyl stehen, $-(CH_2)_nAr$, wobei n für 1 oder 2 steht und Ar für einen unsubstituierten oder ein- oder zweifach substituierten Phenylrest Phenylrest steht, oder $-(CH_2)_nHet$, wobei n für 1, 2 oder 3 steht und Het für einen Pyridylrest oder einen Imidazolylrest steht, bedeutet, oder wenn die Reste $R^3$ und $R^4$ zusammen mit dem diese bindenden Stickstoffatom einen Pyrrolidinring oder einen Piperazinring, der durch Methyl substituiert ist, bilden.

Bevorzugte Verbindungen der allgemeinen Formel I sind weiterhin diejenigen, in denen

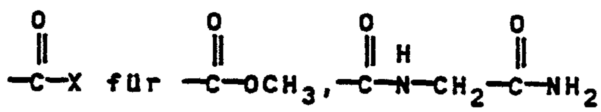
für

oder insbesondere

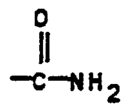

steht.

Eine besonders bevorzugte Verbindung der allgemeinen Formel I ist diejenige, in der $R^2$ für Hydroxymethyl und $R^1$ für $CONH_2$ steht, d.h. das 4-Hydroxymethyl-2-ozyfurazan-3-carbonsäureamid.

Die erfindungsgmäßen Verbindungen der allgemeinen Formel I, in denen einer der Reste $R^1$ und $R^2$ für $-CONR^3R^4$ steht, können beispielsweise dadurch hergestellt werden, daß die literaturbekannte Verbindung der Formel II (siehe Synthesis 1979, S.977) mit einem Oxidationsmittel zur Verbindung der Formel III oder der Formel IV oder zu einem Gemisch der Verbindungen der Formeln III und IV

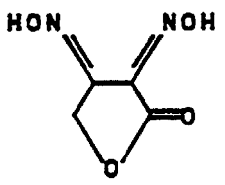

(II)

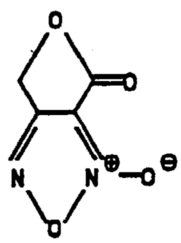

(III)

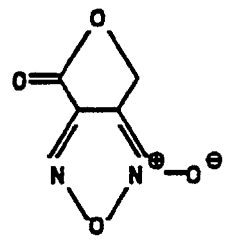

(IV)

oxidiert wird und die Verbindungen der Formel III und/oder IV anschließend mit einem Amin $HNR^3R^4$, worin $R^3$ und $R^4$ wie oben angegeben definiert sind, umgesetzt werden. Als Oxidationsmittel können dabei herkömmliche Reagentien, wie zum Beispiel Halogene, N-Chlor- und N-Bromsuccinimid, Alkali- und Erdalkalihypochlorite, Alkylhypochlorite, wie z.B. tert.-Butylhypochlorit, Blei(Iv)verbindungen, wie beispielsweise Blei(IV)acetat, Eisen(III)-salze, wie beispielsweise rotes Blutlaugensalz, oder nitrose Gase, wie beispielsweise $N_2O_3$ oder $N_2O_4$, eingesetzt werden. Bevorzugte Oxidationsmittel sind Alkali- und Erdalkalihypochlorite und Alkylhypochlorite. Die Umsetzung wird bevorzugt in einem Lösungsmittel, wie beispielsweise Wasser, einem Alkohol, wie beispielsweise Methanol oder Ethanol, einem Ether, einem Ester, wie beispielsweise Ethylacetat, einer Carbonsäure, wie beispielsweise Essigsäure, Methylenchlorid, Chloroform, Cyclohexan, Benzol, Toluol, Chlorbenzol, Dichlorbenzol, DMF oder DMSO oder in einem Lösungsmittelgemisch bei Temperaturen von -10°C bis 50°C, vorzugsweise von -5°C bis 25°C, ausgeführt. Die Verbindungen der Formeln III und/oder IV können auch ohne Isolierung direkt weiter umgesetzt werden.

Die Verbindungen der Formeln III und/oder IV können auch zuerst mit Alkoholen $R^7OH$, wobei $R^7$ die angegebenen Bedeutungen hat, in die erfindungsgemäßen Ester der allgemeinen Formeln Ic und/oder Id umgewandelt werden. Die Alkoholyse kann auch ohne Isolierung der Verbindungen der Formeln III und/oder IV durchgeführt werden, beispielsweise kann die Oxidation der Verbindung der Formel II so durchgeführt werden, daß direkt die Verbindungen der allgemeinen Formeln Ic und/oder Id erhalten werden.

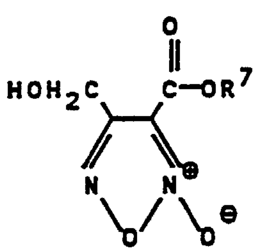

(Ic)

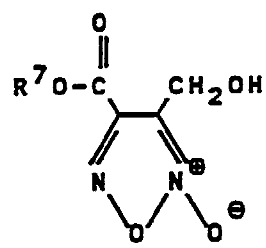

(Id)

Die erfindungsgemäßen Ester der allgemeinen Formeln Ic und/oder Id können gegebenenfalls mit Aminen $R^3R^4NH$ wiederum in erfindungsgemäße Amide überführt werden. Diese Aminolyse wie auch die der Verbindungen der Formeln III und/oder IV kann mit oder ohne ein Lösungsmittel durchgeführt werden. Als Lösungsmittel kommen beispielsweise Wasser, Alkohole, wie Methanol, Ethanol, n- oder i-Propanol, n- oder i-Butanol, Ester, wie Ethyl- oder Isopropylacetat, Ether, wie Diethyl-, Dipropyl-, Dibutylether, tert.-Butylmethylether, Tetrahydrofuran, Dioxan oder Ethylenglykol- oder Diethylenglykol-mono-oder -dialkylether, Kohlenwasserstoffe, wie Toluol oder Xylol, Chlorkohlenwasserstoffe, wie Dichlormethan, Chlorbenzol oder Dichlorbenzol oder beispielsweise Dimethylformamid, N-Methylpyrrolidon oder Dimethylsulfoxid in Frage. Die Reaktionstemperaturen liegen zwischen -10° und 140°C, bevorzugt zwischen 0° und 80°C.

Die Verbindungen der allgemeinen Formel I, in denen einer der Reste $R^1$ und $R^2$ für $-CON^3R^4$ steht, können weiterhin auch dadurch erhalten werden, daß die Verbindung der Formel II zuerst mit Aminen $R^3R^4NH$, worin $R^3$ und $R^4$ wie oben angegebenen definiert sind, zur Verbindung der allgemeinen Formel V

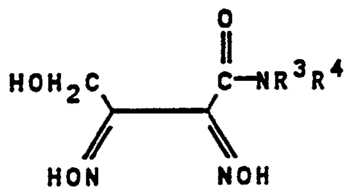

(V)

umgesetzt und diese dann zu den Verbindungen der allgemeinen Formel I oxidiert wird, wobei die bei den vorherigen Verfahrensvarianten beispielhaft angeführten Lösungs- und Oxidationsmittel auch hier beim jeweiligen Reaktionsschritt eingesetzt werden können.

Gegebenenfalls können die nach einem der obenstehenden Verfahren hergestellten erfindungsgemäßen Verbindungen der allgemeinen Formel I durch Modifizierung der Substituenten in weitere erfindungsgemäße Verbindungen der allgemeinen Formel I umgewandelt werden. Beispielsweise kann die Seitenkette $-CO-NH-(CH_2)_mCOOR^5$ durch Umsetzung mit einem Amin $HNR^5R^6$ in die Seitenkette $-CO-NH-(CH_2)_mCONR^5R^6$ umgewandelt werden. Analoges ist mit der Seitenkette $-CO-NH-CH-(Alk)-COOR^5$ möglich.

Verbindungen der allgemeinen Formel I, in denen einer der Reste $R^1$ und $R^2$ für $-CON^3R^4$ steht, können durch Umsetzung von Verbindungen der allgemeinen Formel VI,

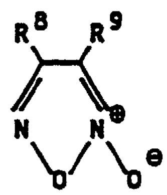

(VI)

worin einer der Reste $R^8$ und $R^9$ für Hydroxymethyl und der andere für eine reaktive Säuregruppe, z.B.

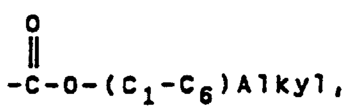, 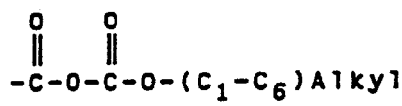,

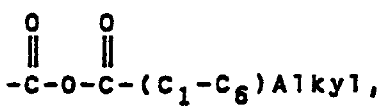, 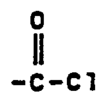 oder 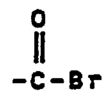,

steht, mit einem Amin $HNR^3R^4$, worin $R^3$ und $R^4$ wie oben angegeben definiert sind, erhalten werden. Vorteilhafterweise wird die Reaktion in Gegenwart einer Base ausgeführt, die entstehende Säuren neutralisiert. Bevorzugte Basen sind Alkalicarbonate, wie Natrium- oder Kaliumhydrogencarbonat oder Natrium- oder Kaliumcarbonat, Alkalihydroxide, wie Natrium-, Kalium- oder Lithiumhydroxid, Alkalialkoholate, wie Natrium- oder Kaliummethylat, Natrium- oder Kaliumethylat oder Kalium-tert.-butylat, Alkalihydride, wie Natrium- oder Kaliumhydrid, Alkaliamide, wie Natriumamid oder Lithiumdiisopropylamid oder organische Basen wie Pyridin oder Triethylamin. Diese Basen werden bevorzugt in molaren Mengen eingesetzt. Als Lösungsmittel kommen je nach Art der reaktiven Säuregruppe beispielsweise Wasser, Alkohole, wie Methanol, Ethanol, n- oder i-Propanol, n- oder i-Butanol, Ether, wie Diethyl-, Dipropyl-, Dibutylether, Tetrahydrofuran, Dioxan oder Ethylenglykol- oder Diethylenglykol-mono-oder -dialkylether, Kohlenwasserstoffe, wie Toluol oder Xylol, Chlorkohlenwasserstoffe, wie Dichlormethan, Chlorbenzol oder Dichlorbenzol, oder beispielsweise Dimethylformamid, N-Methylpyrrolidon oder Dimethylsulfoxid in Frage. Die Reaktionstemperaturen liegen zwischen -10°C und 80°C.

Bei den genannten Reaktionen zur Herstellung der Verbindungen der allgemeinen Formel I fallen diese oder die Zwischenprodukte der allgemeinen Formeln III/IV, Ic/Id und VI in der Regel in Form von Isomerengemischen an, im Falle der Verbindungen der allgemeinen Formel I also als Gemisch der Verbindungen der allgemeinen Formeln Ia und Ib. Diese lassen sich aber durch bekannte Methoden, wie Umkristallisieren oder chromatographische Methoden, insbesondere Säulenchromatographie, trennen. Die Trennung kann also auf der Stufe eines Zwischenproduktes oder auf

der Stufe der erfindungsgemäßen Verbindungen der allgemeinen Formel I erfolgen. Wenn die zu trennenden Verbindungen eine basische oder eine saure Gruppe tragen, kann es vorteilhaft sein, sie erst in ein Salz zu überführen und dann das Gemisch der isomeren Salze zu trennen. Isomerengemische werden auch erhalten, wenn ein reines Isomeres in Substanz oder in einem inerten Lösungsmittel gelöst auf Temperaturen von 50 bis 200°C erhitzt oder bei 0 bis 50°C photolysiert wird. Durch Trennung des so erhaltenen Gemisches ist es somit möglich, ein Isomeres in das andere umzuwandeln.

Erfindungsgemäße Verbindungen der allgemeinen Formel I, die eine basische Gruppe enthalten, können mit anorganischen oder organischen Säuren Säureadditionssalze bilden. Geeignete Säuren für die Bildung pharmakologisch annehmbarer Salze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden. Entsprechend können gegebenenfalls auch Säureadditionssalze, die zur Isomerentrennung aus den Verbindungen der allgemeinen Formel I hergestellt werden, direkt durch Anionenaustausch oder über die freie Base in für die Verwendung der Substanzen gewünschte pharmakologisch annehmbare Säureadditionssalze umgewandelt werden.

Erfindungsgemäße Verbindungen der allgemeinen Formel I, die eine saure Gruppe, beispielsweise eine Carbonsäuregruppe, enthalten, können mit anorganischen oder organischen Basen Salze bilden. Geeignete pharmakologisch annehmbare Salze sind beispielsweise Natriumsalze, Kaliumsalze, magnesiumsalze, Calciumsalze, Ammoniumsalze oder Salze mit organischen Aminen, beispielsweise Ethanolamin oder Aminosäuren.

Die Hydroxymethylfurazancarbonsäurederivate der allgemeinen Formel I und ihre pharmakologisch annehmbaren Salze besitzen wertvolle pharmakologische Eigenschaften, die auf der Bildung von Stickstoffmonoxid und dessen Wirkungen beruhen. Im glatten Muskel führen sie so zu einer Relaxation, und in den Blutplättchen zeigen sie antiadhäsive und antiaggregatorische Wirkungen. Stickstoffmonoxid ist außerdem entscheidend beteiligt bei Lernvorgängen, bei der Regulation von Nierenfunktionen, bei der Immunabwehr oder bei erektilen Dysfunktionen. Die Verbindungen der allgemeinen Formel I können also bei den genannten Indikationen eingesetzt werden, vor allem bei der Bekämpfung bzw. Vorbeugung von Erkrankungen des kardiovaskulären Systems, besonders bei Angina pectoris.

Die pharmakologische Wirkung der Verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Durch kumulative Zugabe der Prüfsubstanz wird ihre relaxierende Wirkung ermittelt. Aus der Konzentrations-Wirkungskurve (Abszisse: -log Konzentration (mol/l) der Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l).

In der folgenden Tabelle sind neben so erhaltenen $IC_{50}$-Werten erfindungsgemäßer Verbindungen die entsprechenden Werte für Molsidomin und Isosorbid-5-mononitrat angegeben, zweier bei der Behandlung der Angina pectoris häufig eingesetzter Wirkstoffe. Gegenüber diesen Substanzen zeichnen sich die erfindungsgemäßen Verbindungen durch eine deutlich verbesserte Wirkung aus.

| | $IC_{50}$ (mol/l) |
|---|---|
| Verbindung 1 I (Beispiel 1) | $8 \cdot 10^{-6}$ |
| Verbindung 3 I (Beispiel 3) | $1,2 \cdot 10^{-5}$ |
| Verbindung des Beispiels 4 I | $7,3 \cdot 10^{-6}$ |
| Verbindung des Beispiels 8 | $8 \cdot 10^{-6}$ |
| Verbindung des Beispiels 18 | $8 \cdot 10^{-6}$ |
| Verbindung des Beispiels 23 | $1,5 \cdot 10^{-5}$ |
| Verbindung des Beispiels 24 | $2 \cdot 10^{-6}$ |
| Verbindung des Beispiels 26 | $1,1 \cdot 10^{-5}$ |
| Molsidomin | $3 \cdot 10^{-4}$ |
| Isosorbid-5-mononitrat | $>1 \cdot 10^{-4}$ |

Die Verbindungen der allgemeinen Formel I und ihre pharmakologisch annehmbaren Salze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine

wirksame Dosis mindestens einer Verbindung der allgemeinen Formel I oder eines Salzes davon neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektions- oder Infusionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Salze und noch andere therapeutisch wirksame Stoffe enthalten.

Derartige andere therapeutisch wirksame Substanzen sind beispielsweise: ß-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Hydroxymethylfurazancarbonsäurederivate der allgemeinen Formel I, ihre pharmakologisch annehmbaren Salze und pharmazeutische Präparate, welche die Verbindungen der allgemeinen Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Darüberhinaus können sie auch zur Behandlung erektiler Dysfunktionen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis in ähnlichen Mengenbereichen, d.h. im allgemeinen ebenfalls bei 0,5 bis 100 mg/Mensch. Die Tagesdosis kann in mehrere, z.B. 2 bis 4, Teilverabreichungen aufgeteilt werden.

**BEISPIELE**

$^1$H-NMR-Spektren sind in $D_6$-DMSO als Lösungsmittel gemessen. Angegeben sind die chemischen Verschiebungen δ in ppm, die Multiplizitäten (s=Singulett, d=Dublett, t=Triplett, m-Multiplett) und die Protonenzahl der Signale.

**1. 4-Hydroxymethyl-N-isopropyl-2-oxyfurazan-3-carbonsäureamid und 4-Hydroxymethyl-N-isopropyl-5-oxyfurazan-3-carbonsäureamid**

a) <u>4-Hydroxy-N-isopropyl-2,3-dioximinobutyramid</u>

9,4 g 3,4-Dioximmo-2-oxotetrahydrofuran werden in 50 ml Methanol suspendiert und mit 4,2 g Isopropylamin versetzt. Die Mischung wird 3 h gerührt und im Eisbad abgekühlt. Der Feststoff wird abgesaugt und aus Methanol umkristallisiert.
Ausbeute: 8,5 g; Fp. 133 - 134°C

b) <u>4-Hydroxymethyl-N-isopropyl-2-oxyfurazan-3-carbonsäureamid und 4-Hydroxymethyl-N-isopropyl-5-oxyfurazan-3-carbonsäureamid</u>

Die Mischung aus 10 g der Verbindung aus Schritt a), 120 ml Methylenchlorid und 22 g Bleitetraacetat wird 5 h gerührt. Die wasserlöslichen Anteile werden dreimal mit je 100 ml Wasser ausgeschüttelt, die organische Phase wird mit Natriumsulfat getrocknet und im Vakuum eingeengt. Das verbleibende Öl wird durch Säulenchromatographie aufgetrennt (Kieselgel, Laufmittel: Methylenchlorid/Methanol 99:1). Es werden erhalten:
Verbindung 1 I : 2-Oxyderivat, Ausbeute 3,6 g (Öl)
$^1$H-NMR: 1,20(d, 6H), 4,08(m, 1H), 4,69(d,2H), 5,77(t, 1H), 8,25 (m, 1H)
Verbindung 1 II: 5-Oxyderivat, Ausbeute 1,8 g (Öl)
$^1$H-N: 1,20(d, 6H), 4,08(m, 1H), 4,57(d, 2H), 5,68(t, 1H), 9,04 (m, 1H)

**2. 4-Hydroxymethyl-2-oxyfurazan-3-carbonsäuremethylester und 4-Hydroxymethyl-5-oxyfurazan-3-carbonsäuremethylester**

In die Mischung aus 2,9 g 3,4-Dioximino-2-oxotetrahydrofuran, 30 ml Methylenchlorid und 10 ml Methanol werden unter Eiskühlung 8,9 g Bleitetraacetat gegeben. Nach zweistündigem Rühren bei Raumtemperatur werden 3 ml Triethylamin zugegeben; es wird weitere 30 min gerührt, mit 30 ml Methylenchlorid verdünnt und zweimal mit je 30 ml Wasser gewaschen. Die organische Phase wird mit Natriumsulfat getrocknet und im Vakuum eingeengt. Der Rückstand wird mit tert.-Butylmethylether verrührt und der erhaltene Feststoff abgesaugt. Zur Trennung der Isomeren wird aus tert.-Butylmethylether umkristallisiert.

Verbindung 2 I: 2-Oxyderivat: Ausbeute 1,9 g; Fp. 92 - 94°C.
$^1$H-NMR: 3,86(s, 3H), 4,70(s, 2H), 5,76 (m, 1H)

Verbindung 2 II: Das 5-Oxyderivat ist in der Umkristallisationsmutterlauge enthalten.

**3. 4-Hydroxymethyl-2-oxyfurazan-3-carbonsäureamid und 4-Hydroxymethyl-5-oxyfurazan-3-carbonsäureamid**

a) <u>4-Hydroxymethyl-2-oxyfurazan-3-carbonsäureamid</u> (Verbindung 3I)

1,9 g 4-Hydroxymethyl-2-oxyfurazan-3-carbonsäuremethylester aus Beispiel 2) werden in 25 ml Methanol mit 7,2 g einer konzentrierten wäßrigen Ammoniaklösung versetzt. Nach 1 h wird die Mischung im Vakuum eingeengt und der Rückstand aus Wasser umkristallisiert.
Ausbeute: 1,1 g; Fp. 128 - 130°C
$^1$H-NMR: 4,72 (d, 2H), 5,73 (t, 1H) 7,85 (m, 1H), 8,41 (m, 1H)

Die Struktur wurde durch eine Röntgenstrukturanalyse bestätigt.

b) <u>4-Hydroxymethyl-5-oxyfurazan-3-carbonsäureamid</u> (Verbindung 3II)

Entsprechend a) wird der in der Umkristallisationsmutterlauge enthaltene 4-Hydroxymethyl-5-ozyfurazan-3-carbonsäuremethylester aus Beispiel 2) mit Ammoniak umgesetzt. Das Produkt wird durch Säulenchromatographie (Kieselgel, Laufmittel Methylenchlorid/Methanol 98:2) gereinigt. Fp. 145 - 147°C.
$^1$H-NMR 4,57 (d, 2H), 5,60 (t, 1H), 8,20 (m, 1H), 8,50 (m, 1H)

In analoger Weise werden erhalten:

**4 I. 4-Hydroxymethyl-N-methyl-2-oxyfurazan-3-carbonsäureamid**

Fp. 104 - 106°C
$^1$H-NMR: 2,80 (s, 3H), 4,72 (s, 2H), 5,70 (m, 1H), 8,41 (m, 1H)

**4 II. 4-Hydroxymethyl-N-methyl-5-oxyfurazan-3-carbonsäureamid**

Fp. 86 - 88°C
$^1$H-NM 2,91 (d, 3H), 4,57 (d, 2H), 5,63 (t, 1H), 9,12 (m, 1H)

**5. 4-Hydroxymethyl-2-oxyfurazan-3-carbonsäure-(4-methylpiperazid)-hydrochlorid**

Fp. 210°C (Zers.)
$^1$H-NMR 2,82 (s, 3H), 3,0 - 4,4 (m, 8H) 4,62 (s, 2H), 9,0-10,0 (m, 2H)

**6. N-(2-Diisopropylaminoethyl)-4-hydroxymethyl-2-oxyfurazan-3-carbonsäureamid-hydrochlorid**

Fp. 189 - 191°C
$^1$H-NMR 1,40 (d, 12H), 3,2 (m, 2H), 3,70 (m, 4H), 4,74 (s, 2H), 5,75 (m, 1H), 8,84 (m, 1H), 10,30 (m, 1H)

**7. N-Butyl-4-hydroxymethyl-2-oxyfurazan-3-carbonsäureamid**

Öl
$^1$H-NMR: 0,91 (t, 3H), 1,30 (m, 2H), 1,45 (m, 2H), 3,26 (m, 2H), 4,72 (s, 2H), 5,75 (m, 1H), 8,45 (m, 1H)

**8. 4-Hydroxymethyl-N-(3-pyridylmethyl)-2-oxyfurazan-3-carbonsäureamid-hydrochlorid**

Fp. 185°C (Zers.)
$^1$H-NMR: 4,68 (d, 2H), 4,72 (s, 2H), 8,0 (m, 1H), 8,51 (m, 1H), 8,80 (m, 1H), 8,90 (s, 1H), 9,30 (m, 1H)

**9. N-Benzyl-4-hydroxymethyl-2-oxyfurazan-3-carbonsäureamid**

Fp. 94 - 96°C
$^1$H-NMR: 4,48 (s, 2H), 4,73 (s, 2H), 5,80 (m, 1H), 7,30 (m, 5H), 8,85 (m, 1H)

**10. N-Carbamoylmethyl-4-hydroxymethyl-2-oxyfurazan-3-carbonsäureamid**

Fp. 186 - 188°C
$^1$H-NMR: 3,85 (d, 2H), 4,74 (t, 2H), 5,73 (t, 1H), 7,20 (m, 1H), 7,50 (m, 1H), 8,60 (m, 1H)

**11. 4-Hydroxymethyl-2-oxyfurazan-3-carbonsäurepyrrolidid**

Öl
$^1$H-NMR: 1,85 (m, 4H), 3,47 (m, 4H), 4,60 (d, 2H), 5,78 (t, 1H)

**12. 4-Hydroxymethyl-N-isobutyl-2-oxyfurazan-3-carbonsäureamid**

Öl
$^1$H-NMR: 0,89 (d, 6H), 1,84 (m, 1H), 3,12 (m, 2H), 4,72 (d, 2H), 5,77 (t, 1H)

**13. 4-Hydroxymethyl-N-(2-hydroxyethyl)-2-oxyfurazan-3-carbonsäureamid**

Fp. 69-71°C
$^1$H-NMR: 3,36 (m, 2H); 3,53 (m, 2H); 4,72 (d, 2H); 4,86 (t, 1H); 5,72 (t, 1H); 8,40 (m, 1H)

**14. 4-Hydroxymethyl-N-(2-diisopropylaminoethyl)-5-oxyfurazan-3-carbonsäureamid-hydrochlorid**

Fp. 167-170°C
$^1$H-NMR: 1,35 (q, 12H); 3,20 (m, 2H); 3,69 (m, 4H); 4,57 (s, 2H); 5,69 (m, 1H); 9,48 (m, 1H); 10,23 (m, 1H)

**15. 4-Hydroxymethyl-N-(3-(imidazol-1-yl)-propyl)-5-oxyfurazan-3-carbonsäureamid**

Fp. 145-147°C
$^1$H-NMR: 1,95 (m, 2H); 3,25 (m, 2H), 4,00 (m, 2H); 4,54 (s, 2H); 5,71 (m, 1H); 6,87 (s, 1H); 7,18 (s, 1H); 7,62 (s, 1H); 9,27 (m, 1H)

**16. 4-Hydroxymethyl-N-(2-hydroxyethyl)-5-oxyfurazan-3-carbonsäureamid**

Fp. Öl
$^1$H-NMR: 3,32 (m, 2H); 3,52 (m, 2H); 4,55 (d, 2H); 4,70 (m, 1H); 5,67 (t, 1H); 9,09 (m, 1H)

**17. 4-Hydroxymethyl-N,N-dimethyl-2-oxyfurazan-3-carbonsäureamid**

Fp. Öl
$^1$H-NMR: 2,98 (d, 6H); 4,58 (d, 2H); 5,84 (t, 1H)

**18. 4-Hydroxymethyl-N-(2-methoxyethyl)-2-oxyfurazan-3-carbonsäureamid**

Fp. Öl
$^1$H-NHR: 3,26 (s, 3H); 3,47 (s, 4H); 4,73 (s, 2H); 5,77 (m, 1H); 8,48 (m, 1H)

**19. 4-Hydroxymethyl-2-oxyfurazan-3-carbonsäureisopropylester**

Öl
$^1$H-NMR: 1,30 (d, 6H); 4,69 (d, 2H); 5,17 (m, 1H); 5,70 (t, 1H)

**20. 4-Hydroxymethyl-N-(3-(imidazol-1-yl)-propyl)-2-oxyfurazan-3-carbonsäureamid**

Fp. 122-125°C
$^1$H-NMR: 1,93 (m, 2H); 3,23 (m, 2H); 4,00 (m, 2H); 4,72 (s, 2H); 5,89 (m, 1H); 6,90 (s, 1H); 7,18 (s, 1H); 7,63 (s, 1H); 8,60 (m, 1H)

**21. 4-Hydroxymethyl-N-(3-hydroxypropyl)-5-oxyfurazan-3-carbonsäureamid**

Öl
$^1$H-NMR: 1,69 (m, 2H); 3,33 (m, 2H); 3,47 (m, 2H); 4,52 (m, 3H); 5,65 (t, 1H); 9,10 (m, 1H)

**22. 4-Hydroxymethyl-N-(3-pyridylmethyl)-5-oxyfurazan-3-carbonsäureamid**

Fp. 154-156°C
$^1$H-NMR: 4,51 (d, 2H); 4,59 (d, 2H); 5,63 (t, 1H); 7,39 (m, 1H); 7,75 (m, 1H); 8,49 (m, 1H); 8,59 (s, 1H); 9,78 (m, 1H)

**23. 4-Hydroxymethyl-N-((S)-1-phenylethyl)-2-oxyfurazan-3-carbonsäureamid**

Fp. Öl
$\alpha_D^{20}$ = +17,5 (c = 5,472, MeOH)
$^1$H-NMR: 1,48 (d, 3H); 4,69 (d, 2H); 5,10 (m, 1H); 5,87 (t, 1H); 7,33 (m, 5H); 8,84 (d, 1H)

**24. 4-Hydroxymethyl-N-((R)-1-phenylethyl)-2-oxyfurazan-3-carbonsäureamid**

Öl
$\alpha_D^{20}$ = -16,7 (c = 5,618, MeOH)
$^1$H-NMR: 1,48 (d, 3H); 4,69 (d, 2H); 5,10 (m, 1H); 5,87 (t, 1H); 7,33 (m, 5H); 8,84 (d, 1H)

**25. 4-Hydroxymethyl-N-octadecyl-2-oxyfurazan-3-carbonsäureamid**

Fp. 75-77°C
$^1$H-NMR: 0,6-1,6 (m, 35H); 3,30 (m, 2H); 4,70 (m, 2H); 5,76 (m, 1H); 8,50 (m, 1H)

**26. 4-Hydroxymethyl-N-[2-(4-sulfamoyl-phenyl)-ethyl]-2-oxyfurazan-3-carbonsäureamid**

Fp. 152-154°C
$^1$H-NMR: 2,90 (m, 2H); 3,53 (m, 2H); 4,72 (s, 2H); 5,80 (m, 1H) 7,23 (m, 2H); 7,42 (d, 2H); 7,77 (d, 2H); 8,57 (m, 1H)

**27. 4-Hydroxymethyl-2-oxyfurazan-3-carbonsäuremethylester**

a ) 3,4-Dioximino-2-oxotetrahydrofuran

In die Mischung aus 49 g Tetronsäure, 43,4 g konzentrierter Salzsäure und 100 ml Wasser wird unter Eiskühlung eine Lösung von 30,4 g Natriumnitrit in 60 ml Wasser getropft. Die Mischung wird 30 min nachgerührt und dann mit einer Lösung von 30,6 g Hydrozylamin-hydmchlorid in 60 ml Wasser versetzt. Unter Eiskühlung wird noch 3 Stunden nachgerührt und das Produkt abgesaugt, mit Wasser gewaschen und trocken gesaugt.
Ausbeute: 76,3 g (feucht)
Fp. 160 -180°C (Zers.)

3,4-Dioximino-2-oxotetrahydrofuran wird auch aus 4-tert.Butoxyacetessigsäureethylester durch Behandlung mit wäßriger Schwefelsäure oder Salzsäure, Nitrosierung der intermediär gebildeten Tetronsäure und Reaktion mit Hydroxylamin-hydrochlorid in 60% Ausbeute erhalten.

b) 4-Hydroxymethyl-2-oxyfurazan-3-carbonsäuremethylester

In die im Eisbad gekühlte Mischung aus 27 g 3,4-Dioximino-2-oxotetrahydrofuran und Methanol (210 ml) wird eine Lösung von 18,9 g tert.-Butylhypochlorit in 25 ml Ethylacetat rasch zugetropft. Anschließend wird Kaliumacetat (18,5 g) in kleinen Portionen unter Kühlung zugefügt und das Gemisch im sich allmählich auf Raumtemperatur erwärmenden Eisbad über Nacht gerührt. Die Mischung wird im Vakuum eingeengt, der Rückstand in Wasser aufgenommen und das Produkt mit Ethylacetat extrahiert. Nach dem Trocknen und Einengen der Ethylacetatphase wird aus Isopropylacetat umkristallisiert.
Ausbeute: 14,7 g
Fp. 97-98°C

Nach demselben Verfahren wird 4-Hydroxymethyl-2-oxyfurazan-3-carbonsäuremethylester auch erhalten, wenn anstelle der Lösung von tert.-Butylhypochlorit in Ethylacetat festes 70prozentiges Calciumhypochlorit verwendet wird.

**28. 4-Hydroxymethyl-N-cyclohexyl-2-oxyfurazan-3-carbonsäureamid**

Fp. 97-98°C
$^1$H-NMR: 1,10-1,85 (m, 10H); 3,74 (m, 1H); 4,72 (s, 2H); 5,82 (m, 1H); 8,25 (m, 1H)

**29. 4-Hydroxymethyl-N-ethoxycarbonylmethyl-2-oxyfurazan-3-carbonsäureamid**

Fp. Öl
$^1$H-NMR: 1,23 (t, 3H); 4,08 (d, 2H); 4,15 (q, 2H); 4,74 (d, 2H); 5,73 (t, 1H); 8,81 (m, 1H)

**30. 4-Hydroxymethyl-N-(2-acetylamino-ethyl)-2-oxyfurazan-3-carbonsäureamid**

Fp. 126-128°C
$^1$H-NMR: 1,79 (s, 1H); 3,20 (m, 2H); 3,33 (m, 2H); 4,75 (s, 2H); 5,76 (m, 1H); 7,80 (m, 1H); 8,51 (m, 1H)

**31. 4-Hydroxymethyl-N-allyl-2-oxyfurazan-3-carbonsäureamid**

Fp. Öl
$^1$H-NMR: 3,90 (s, 2H); 4,76 (s, 2H); 5,17 (m, 2H); 5,83 (m, 1H) 5,78 (m, 1H); 8,67 (m, 1H)

**32. 4-Hydroxymethyl-N-(1-ethoxycarbonyl-ethyl)-2-oxyfurazan-3-carbonsäureamid**

Fp. Öl
$^1$H-NMR: 1,32 (t, 3H); 1,40 (d, 3H); 4,32 (q, 2H); 4,50 (m, 1H); 4,73 (d, 2H); 5,71 (t, 1H); 8,81 (m, 1H)

**33. 4-Hydroxymethyl-N-[3-(2-oxo-pyrrolidin-1-yl)-propyl]-2-oxyfurazan-3-carbonsäureamid**

Fp. 120-122°C
$^1$H-NMR: 1,65 (m, 2H); 1,92 (m, 2H); 2,22 (m, 2H); 3,21 (m, 4H); 3,38 (m, 2H); 4,74 (s, 2H); 5,73 (m, 1H); 8,55 (m, 1H)

**34. 4-Hydroxymethyl-N-[2-(3,4-dimethoxyphenyl)-ethyl]-2-oxyfurazan-3-carbonsäureamid**

Fp. 128-130°C
$^1$H-NMR: 2,77 (t, 2H); 3,46 (t, 2H); 3,73 (s, 3H); 3,74 (s, 3H); 4,70 (s, 2H); 5,72 (m, 1H); 6,70-6,91 (m, 3H); 8,44 (m, 1H)

In den nachfolgenden Beispielen A bis F werden pharmazeutische Präparate beschrieben.

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | pro ml |
|---|---|
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

Emulsion, enthaltend 3 mg Wirkstoff pro 5 ml

| | pro 100 ml Emulsion |
|---|---|
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

Rektale Arzneiform, enthaltend 4 mg Wirkstoff pro Suppositorium

| | pro Suppositorium |
|---|---|
| Wirkstoff | 4 mg |
| Suppositoriengrundmasse | ad 2 g |

Beispiel E

Tabletten, enthaltend 2 mg Wirkstoff pro Tablette

| | pro Tablette |
|---|---|
| Wirkstoff | 2 mg |
| Lactat (feingemahlen) | 2 mg |
| Maisstärke (weiß) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboxymethylstärke | 25 mg |
| | 311 mg |

Beispiel F

Dragees, enthaltend 1 mg Wirkstoff pro Dragee

| | pro Dragee |
|---|---|
| Wirkstoff | 1 mg |
| Maisstärke | 100 mg |
| Lactose | 60 mg |
| sec.Calciumphosphat | 30 mg |
| lösliche Stärke | 3 mg |
| Magnesiumstearat | 2 mg |
| kolloidale Kieselsäure | 4 mg |
| | 200 mg |

**Patentansprüche**

**1.** Hydroxymethylfurazancarbonsäurederivate der allgemeinen Formel I

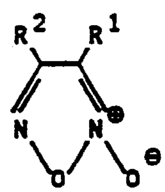

(I)

worin einer der Reste $R^1$ und $R^2$ für Hydroxymethyl und der andere für

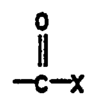

steht, wobei

X für $NR^3R^4$ oder OH oder $OR^7$ steht;
$R^3$ und $R^4$ unabhängig voneinander Wasserstoff, $(C_1\text{-}C_{20})$-Alkyl, 1-Phenyl-$(C_2\text{-}C_4)$-alkyl, 2-Phenyl-$(C_3\text{-}C_4)$-alkyl, $(C_3\text{-}C_6)$-Alkenyl, $(C_3\text{-}C_7)$-Cycloalkyl, $-(CH_2)_n-NR^5R^6$, $-(CH_2)_n-OR^5$, $-(CH_2)_m-COOR^5$, $-CH(Alk)-COOR^5$, $-(CH_2)_m-CONR^5R^6$, $-CH(Alk)-CONR^5R^6$,

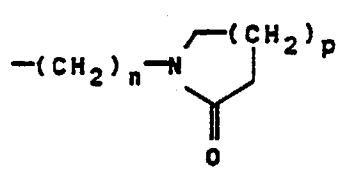 ,

$-(CH_2)_n-NR^5$ (COAlk), $-(CH_2)_n$-Ar oder $-(CH_2)_n$-Het bedeuten oder $R^3$ und $R^4$ zusammen mit dem diese bindenden Stickstoffatom einen Heterocyclus bilden, der auch durch $(C_1\text{-}C_6)$-Alkyl, $(C_3\text{-}C_7)$-Cycloalkyl, $(C_1\text{-}c_4)$-Alkoxy, Amino, $(C_1\text{-}C_4)$-Alkylamino, Di($(C_1\text{-}C_4)$-alkyl)amino, Hydroxy, Acetoxy, Benzyl, Phenethyl oder Ar ein- oder mehrfach substituiert sein kann;
$R^5$ und $R^6$ unabhängig voneinander Wasserstoff, $(C_1\text{-}C_6)$-Alkyl, $(C_3\text{-}C_6)$-Alkenyl, $(C_3\text{-}C_7)$-Cycloalkyl, Benzyl, Phenethyl oder Ar bedeuten;
$R^7$ $(C_1\text{-}C_6)$-Alkyl, $(C_3\text{-}C_7)$-Cycloalkyl, Benzyl, Phenyl oder durch $(C_1\text{-}C_4)$-Alkyl, Fluor, Chlor oder Nitro ein- oder mehrfach substituiertes Phenyl bedeutet;

Alk $(C_1-C_6)$-Alkyl bedeutet;
Ar einen Arylrest mit 6 bis 12 C-Atomen, der auch durch $(C_1-C_4)$-Alkyl, $(C_1-C_4)$-Alkoxy, AminO, $(C_1-C_4)$-Alkylamino, Di($(C_1-C_4)$-alkyl)amino, $(C_1-C_6)$-Alkanoylamino, Sulfamoyl, Fluor, Chlor, Brom, Hydroxy, Acetoxy, Nitro, Trifluormethyl oder Cyano ein- oder mehrfach substituiert sein kann, bedeutet;
Het einen heterocyclischen Rest mit 1 bis 3 Heteroatomen, der auch durch $(C_1-C_4)$-Alkyl, $(C_1-C_4)$-Alkoxy, Amino, $(C_1-C_4)$-Alkylamino, Di($(C_1-C_4)$-alkyl)amino, $(C_1-C_6)$-Alkanoylamino, Fluor, Chlor, Brom, Hydroxy, Acetoxy, Nitrc, Cyano oder Ar ein- oder mehrfach substituiert sein kann, bedeutet;
n für 0, 1, 2, 3 oder 4,
m für 1, 2, 3 oder 4,
p für 1, 2 oder 3 steht;

sowie deren pharmakologisch annehmbare Salze.

2. Hydroxymethylfurazancarbonsäurederivate der allgemeinen Formel I gemäß Anspruch 1, dadurch gekennzeichnet, daß X für $NR^3R^4$ steht und daß einer der Reste $R^3$ und $R^4$ für Wasserstoff steht und der andere Rest eine der in Anspruch 1 gegebenen Definitionen besitzt, oder daß $R^3$ und $R^4$ zusammen mit dem diese bindenden Stickstoffatom einen Heterocyclus bilden, der auch durch $(C_1-C_6)$-Alkyl, $(C_3-C_7)$-cycloalkyl, $(C_1-C_4)$-Alkoxy, Amino, $(C_1-C_4)$-Alkulamino, Di($(C_1-C_4)$-alkyl)amino, Hydroxy, Acetoxy, Benzyl, Phenethyl oder Ar ein- oder mehrfach substituiert sein kann.

3. Hydroxymethylfurazancarbonsänrederivate der allgemeinen Formel I gemäß Anspruch 1 und/oder 2, dadurch gekennzeichnet, daß X für $NR^3R^4$ steht und daß einer der Reste $R^3$ und $R^4$ für Wasserstoff steht und der andere für Wasserstoff, $(C_1-C_6)$-Alkyl, $-(CH_2)_nNR^5R^6$, wobei n 1, 2, 3 oder 4 bedeutet und $R^5$ und $R^6$ $(C_1-C_6)$-Alkyl bedeuten, $-(CH_2)_nOR^5$, wobei n 2, 3 oder 4 bedeutet und $R^5$ Wasserstoff oder $(C_1-C_4)$-Alkyl bedeutet, $-(CH_2)_nAr$, wobei n 0, 1, 2, 3 oder 4 bedeutet und Ar unsubstituiertes oder ein- oder mehrfach substituiertes Phenyl bedeutet, oder $-(CH_2)_nHet$, wobei n 1, 2, 3 oder 4 bedeutet und Het einen heterocyclischen Ring bedeutet, der als Heteroatome 1 oder 2 Stickstoffatome oder 1 Stickstoffatom und 1 Sauerstoffatom enthält und durch Methylgruppen ein- oder mehrfach substituiert sein kann, steht, oder daß $R^3$ und $R^4$ zusammen mit dem diese bindenden Stickstoffatom einen Heterocyclus bilden, der auch durch Methylgruppen ein- oder mehrfach substituiert sein kann.

4. Hydroxymethylfurazancarbonsäurederivate der allgemeinen Formel I gemäß einem oder mehreren der Ansprüche 1 bis 3, dadurch gekennzeichnet, daß X für $NR^3R^4$ steht, und daß einer der Reste $R^3$ und $R^4$ für Wasserstoff steht und der andere für Wasserstoff, $(C_1-C_6)$)-Alkyl, $-(CH_2)_nNR^5R^6$, wobei n 1, 2 oder 3 bedeutet und $R^5$ und $R^6$ $(C_1-C_6)$-Alkyl bedeuten, $-(CH_2)_nAr$, wobei n 1 oder 2 bedeutet und Ar unsubstituiertes oder ein- oder zweifach substituiertes Phenyl bedeutet, oder $-(CH_2)_nHet$, wobei n 1, 2 oder 3 bedeutet und Het Pyridyl oder Imidazolyl bedeutet, steht, oder daß die Reste $R^3$ und $R^4$ zusammen mit diese bindenden Stickstoffatom einen Pyrrolidinring oder einen Piperazinring, der durch Methyl substituiert ist, bilden.

5. Hydroxymethylfurazancarbonsäurederivate der allgemeinen Formel I gemäß Anspruch 1, dadurch gekennzeichnet, daß einer der Reste $R^1$ und $R^2$ für Hydroxymethyl und der andere für

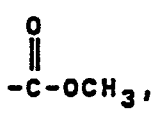,
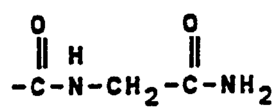

oder bevorzugt

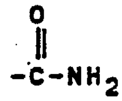

steht.

6. Hydroxymethylfurazancarbonsäurederivate der allgemeinen Formel I gemäß einem oder mehreren der Ansprüche 1 bis 5, dadurch gekennzeichnet, daß $R^1$ für Hydroxymethyl steht.

**7.** Hydroxymethylfurazancarbonsäurederivate der allgemeinen Formel I gemäß einem oder mehreren der Ansprüche 1 bis 5, dadurch gekennzeichnet, daß $R^2$ für Hydroxymethyl steht.

**8.** 4-Hydroxymethyl-2-oxyfurazan-3-carbonsäureamid.

**9.** Verfahren zur Herstellung von Hydroxymethylfurazancarbonsäurederivaten der allgemeinen Formel I gemäß einem oder mehreren der Ansprüche 1 bis 8 und ihrer pharmakologisch annehmbaren Salze, dadurch gekennzeichnet, daß die Verbindung der Formel II zur Verbindung der Formel III

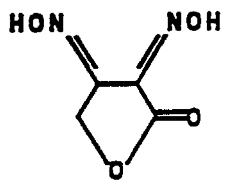
(II)

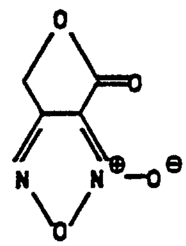
(III)

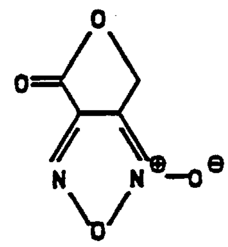
(IV)

oder der Formel IV oder zu einem Gemisch der Verbindungen der Formeln III und IV oxidiert wird und die Verbindungen der Formeln III und/oder IV mit einem Amin $R^3R^4NH$, worin $R^3$ und $R^4$ wie in den Ansprüchen 1 bis 8 angegeben definiert sind, zu Verbindungen der allgemeinen Formel I, in der einer der Reste $R^1$ und $R^2$ für $-CONR^3R^4$ steht, umgesetzt werden,
oder die Verbindungen der Formeln III und/oder IV mit einem Alkohol $R^7OH$, worin $R^7$ wie in Anspruch 1 angegeben definiert ist, zu Verbindungen der allgemeinen Formeln Ic oder Id

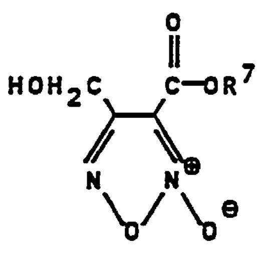
(Ic)

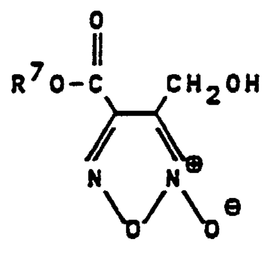
(Id)

oder zu einem Gemisch der Verbindungen der allgemeinen Formeln Ic und Id umgesetzt werden und gegebenenfalls die Verbindungen der allgemeinen Formeln Ic und/oder Id mit einem Amin $R^3R^4NH$ in Verbindungen der allgemeinen Formel I, in der einer der Reste $R^1$ und $R^2$ für $-CONR^3R^4$ steht, überführt werden,
oder daß die Verbindung der Formel II mit einem Amin $R^3R^4NH$, worin $R^3$ und $R^4$ wie in den Ansprüchen 1 bis 8 angegeben definiert sind, zur Verbindung der allgemeinen Formel V

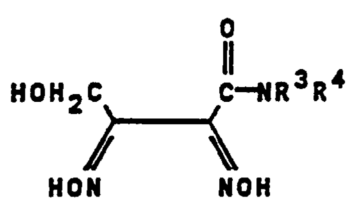
(V)

umgesetzt wird und diese zu Verbindungen der allgemeinen Formel I, in der einer der Reste $R^1$ und $R^2$ für $CON^3R^4$ steht, oxidiert wird,

und daß gegegebenenfalls das Gemisch der isomeren Verbindungen der allgemeinen Formel I oder das Isomerengemisch der Verbindungen der Formeln III und IV oder der allgemeinen Formeln Ic und Id oder Salze dieser Verbindungen nach an sich bekannten Methoden getrennt werden,
und daß gegebenenfalls eine Verbindung der allgemeinen Formel I oder ein Salz davon in ein pharmakologisch annehmbares Salz überführt wird.

**10.** Hydroxymethylfurazancarbonsäurederivate der allgemeinen Formel I gemäß einem oder mehreren der Ansprüche 1 bis 8 und deren pharmakologisch annehmbare Salze zur Anwendung als Arzneimittel.

**11.** Hydroxymethylfurazancarbonsäurederivate der allgemeinen Formel I gemäß einem oder mehreren der Ansprüche 1 bis 8 und deren pharmakologisch annehmbare Salze zur Bekämpfung und Vorbeugung von Erkrankungen des kardiovaskulären Systems, insbesondere der Angina pectoris, oder zur Behandlung erektiler Dysfunktionen.

**12.** Pharmazeutisches Präparat, dadurch gekennzeichnet, daß es ein oder mehrere der Hydroxymethylfurazancarbonsäurederivate der allgemeinen Formel I gemäß einem oder mehreren der Ansprüche 1 bis 8 und/oder ein oder mehrere pharmakologisch annehmbare Salze davon als Wirkstoff zusammen mit pharmazeutisch annehmbaren Träger- und Zusatzstoffen und gegebenenfalls noch einen oder mehrere andere pharmakologische Wirkstoffe enthält.

## Claims

**1.** Hydroxymethylfurazancarboxylic acid derivatives of the formula I

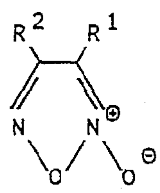

(I)

in which one of the radicals $R^1$ and $R^2$ represents hydroxymethyl and the other represents

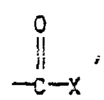

where

X represents $NR^3R^4$ or OH or $OR^7$;
$R^3$ and $R^4$, independently of each other, denote hydrogen, $(C_1-C_{20})$-alkyl, 1-phenyl-$(C_2-C_4)$-alkyl, 2-phenyl-$(C_3-C_4)$-alkyl,
$(C_3-C_6)$-alkenyl, $(C_3-C_7)$-cycloalkyl, $-(CH_2)_n-NR^5R^6$, $-(CH_2)_n-OR^5$, $-(CH_2)_m-COOR^5$, $-CH(Alk)-COOR^5$, $-(CH_2)_m-CONR^5R^6$, $-CH(Alk)-CONR^5R^6$,

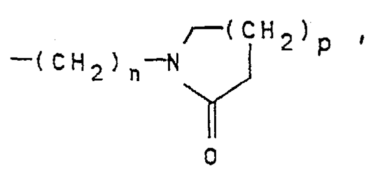

$-(CH_2)_n-NR^5(COAlk)$, $-(CH_2)_n-Ar$ or $-(CH_2)_n-Het$, or $R^3$ and $R^4$, together with the nitrogen atom linking them, form a heterocycle, which can also be substituted once or more than once by $(C_1-C_6)$-alkyl, $(C_3-C_7)$-cycloalkyl, $(C_1-C_4)$-alkoxy, amino, $(C_1-C_4)$-alkylamino, di $((C_1-C_4)$-alkyl) amino, hydroxyl, acetoxy, benzyl, phenethyl or Ar;

$R^5$ and $R^6$, independently of each other, denote hydrogen, $(C_1$-$C_6)$-alkyl, $(C_3$-$C_6)$-alkenyl, $(C_3$-$C_7)$-cycloalkyl, benzyl, phenethyl or Ar;
$R^7$ denotes $(C_1$-$C_6)$-alkyl, $(C_3$-$C_7)$-cycloalkyl, benzyl or phenyl, or phenyl which is substituted once or more than once by $(C_1$-$C_4)$-alkyl, fluorine, chlorine or nitro;
Alk denotes $(C_1$-$C_6)$-alkyl;
Ar denotes an aryl radical having 6 to 12 C atoms which can also be substituted once or more than once by $(C_1$-$C_4)$-alkyl, $(C_1$-$C_4)$-alkoxy, amino, $(C_1$-$C_4)$-alkylamino, di($(C_1$-$C_4)$-alkyl)amino, $(C_1$-$C_6)$-alkanoylamino, sulphamoyl, fluorine, chlorine, bromine, hydroxyl, acetoxy, nitro, trifluoromethyl or cyano;
Het denotes a heterocyclic radical having 1 to 3 heteroatoms, which can also be substituted once or more than once by $(C_1$-$C_4)$-alkyl, $(C_1$-$C_4)$-alkoxy, amino, $(C_1$-$C_4)$-alkylamino, di ($(C_1$-$C_4)$-alkyl)amino, $(C_1$-$C_6)$-alkanoylamino, fluorine, chlorine, bromine, hydroxyl, acetoxy, nitro, cyano or Ar;
n represents 0, 1, 2, 3 or 4,
m represents 1, 2, 3 or 4,
p represents 1, 2 or 3;

and pharmacologically acceptable salts thereof.

**2.** Hydroxymethylfurazancarboxylic acid derivatives of the formula I according to Claim 1, characterized in that X represents $NR^3R^4$, and in that one of the radicals $R^3$ and $R^4$ represents hydrogen and the other radical possesses one of the definitions given in Claim 1, or in that $R^3$ and $R^4$, together with the nitrogen atom linking them, form a heterocycle, which can also be substituted once or more than once by $(C_1$-$C_6)$-alkyl, $(C_3$-$C_7)$-cycloalkyl, $(C_1$-$C_4)$-alkoxy, amino, $(C_1$-$C_4)$-alkylamino, di ($(C_1$-$C_4)$-alkyl) amino, hydroxyl, acetoxy, benzyl, phenethyl or Ar.

**3.** Hydroxymethylfurazancarboxylic acid derivatives of the formula I according to Claim 1 and/or 2, characterized in that X represents $NR^3R^4$, and in that one of the radicals $R^3$ and $R^4$ represents hydrogen and the other represents hydrogen, $(C_1$-$C_6)$-alkyl, $-(CH_2)_nNR^5R^6$, where n denotes 1, 2, 3 or 4 and $R^5$ and $R^6$ denote $(C_1$-$C_6)$-alkyl, $-(CH_2)_nOR^5$, where n denotes 2, 3 or 4 and $R^5$ denotes hydrogen or $(C_1$-$C_4)$-alkyl, $-(CH_2)_nAr$, where n denotes 0, 1, 2, 3 or 4 and Ar denotes unsubstituted or mono- or polysubstituted phenyl, or $-(CH_2)_nHet$, where n denotes 1, 2, 3 or 4 and Het denotes a heterocyclic ring, which contains, as heteroatoms, 1 or 2 nitrogen atoms or 1 nitrogen atom and 1 oxygen atom, and which can be substituted once or more than once by methyl groups, or in that $R^3$ and $R^4$, together with the nitrogen atoms linking them, form a heterocycle which can also be substituted once or more than once by methyl groups.

**4.** Hydroxymethylfurazancarboxylic acid derivatives of the formula I according to one or more of Claims 1 to 3, characterized in that X represents $NR^3R^4$, and in that one of the radicals $R^3$ and $R^4$ represents hydrogen and the other represents hydrogen, $(C_1$-$C_6)$-alkyl, $-(CH_2)_nNR^5R^6$, where n denotes 1, 2 or 3 and $R^5$ and $R^5$ denote $(C_1$-$C_6)$-alkyl, $-(CH_2)_nAr$, where n denotes 1 or 2 and Ar denotes unsubstituted or mono- or disubstituted phenyl, or $-(CH_2)_nHet$, where n denotes 1, 2 or 3 and Het denotes pyridyl or imidazolyl, or in that the radicals $R^3$ and $R^4$, together with the nitrogen atom linking them, form a pyrrolidine ring or a piperazine ring which is substituted by methyl.

**5.** Hydroxymethylfurazancarboxylic acid derivatives of the formula I according to Claim 1, characterized in that one of the radicals $R^1$ and $R^2$ represents hydroxymethyl and the other represents

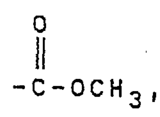,
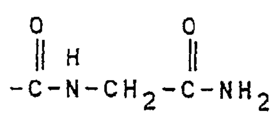

or, preferably,

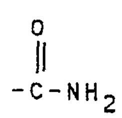

**6.** Hydroxymethylfurazancarboxylic acid derivatives of the formula I according to one or more of Claims 1 to 5, char-

acterized in that $R^1$ represents hydroxymethyl.

7. Hydroxymethylfurazancarboxylic acid derivatives of the formula I according to one or more of Claims 1 to 5, characterized in that $R^2$ representd hydroxymethyl.

8. 4-Hydroxymethyl-2-oxyfurazan-3-carboxamide.

9. Process for preparing hydroxymethylfurazancarboxylic acid derivatives of the formula I according to one or more of Claims 1 to 8, and their pharmacologically acceptable salts, characterized in that the compound of the formula II is oxidized to the compound of the formula III

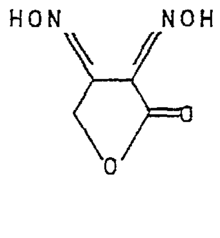
(II)

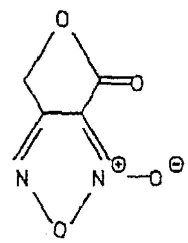
(III)

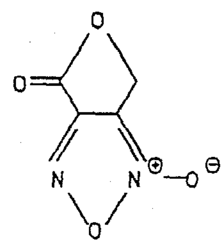
(IV)

or of the formula IV, or to a mixture of the compounds of the formulae III and IV, and the compounds of the formulae III and/or IV are reacted with an amine $R^3R^4NH$, in which $R^3$ and $R^4$ are defined as indicated in Claims 1 to 8, to give compounds of the formula I in which one of the radicals $R^1$ and $R^2$ represents $-CONR^3R^4$,
or the compounds of the formulae III and/or IV are reacted with an alcohol $R^7OH$, in which $R^7$ is defined as indicated in Claim 1, to give compounds of the formulae Ic or Id

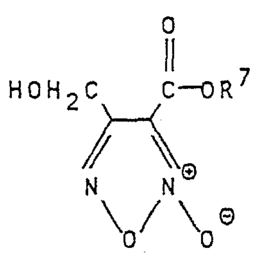
(Ic)

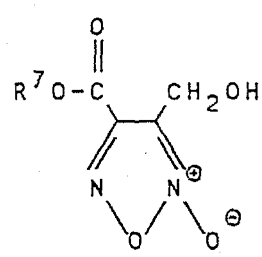
(Id)

or to give a mixture of the compounds of the formulae Ic and Id, and, where appropriate, the compounds of the formulae Ic and/or Id are converted with an amine $R^3R^4NH$ into compounds of the formula I in which one of the radicals $R^1$ and $R^2$ represents $-CONR^3R^4$,
or in that the compound of the formula II is reacted with an amine $R^3R^4NH$, in which $R^3$ and $R^4$ are defined as indicated in Claims 1 to 8, to give the compound of the formula V

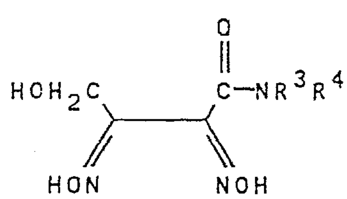
(V)

and this latter compound is oxidized to give compounds of the formula I in which one of the radicals $R^1$ and $R^2$

represents $CON^3R^4$,

and in that, where appropriate, the mixture of the isomeric compounds of the formula I, or the isomeric mixture of the compounds of the formulae III and IV or of the formulae Ic and Id or salts of these compounds, are resolved by methods which are known per se,

and in that, where appropriate, a compound of the formula I, or a salt thereof, is converted into a pharmacologically acceptable salt.

**10.** Hydroxymethylfurazancarboxylic acid derivatives of the formula I according to one or more of Claims 1 to 8, and their pharmacologically acceptable salts, for use as medicaments.

**11.** Hydroxymethylfurazancarboxylic acid derivatives of the formula I according to one or more of Claims 1 to 8, and their pharmacologically acceptable salts, for controlling and preventing disorders of the cardiovascular system, in particular angina pectoris, or for treating erectile dysfunctions.

**12.** Pharmaceutical preparation, characterized in that it contains one or more of the hydroxymethylfurazancarboxylic acid derivatives of the formula I according to one or more of Claims 1 to 8, and/or one or more pharmacologically acceptable salts thereof, as the active compound, together with pharmaceutically acceptable carrier substances and additives and, where appropriate, one or more different pharmacological active compounds as well.

## Revendications

**1.** Dérivés de l'acide hydroxyméthyl-furazanne-carboxylique de formule générale I

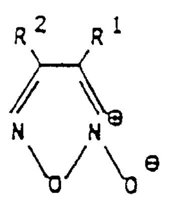

(I)

dans laquelle un des groupes $R^1$ et $R^2$ représente un groupe hydroxyméthyle et l'autre un groupe

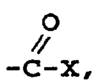

X représentant un groupe $NR^3R^4$ ou OH ou $OR^7$;

$R^3$ et $R^4$ étant indépendamment l'un de l'autre un atome d'hydrogène, un groupe alkyle en $C_1$-$C_{20}$, 1-phényl-alkyle en $C_2$-$C_4$, 2-phényl-alkyle en $C_3$-$C_4$, alcényle en $C_3$-$C_6$, cycloalkyle en $C_3$-$C_7$, $-(CH_2)_n-NR^5R^6$, $-(CH_2)_n-OR^5$, $-(CH_2)_m-COOR^5$, -CH(alk)-$COOR^5$, $-(CH_2)_m-CONR^5R^6$, -CH-(alk)-$CONR^5R^6$

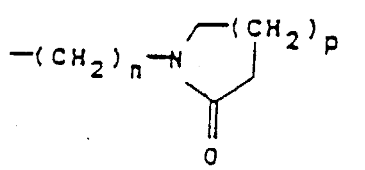

$-(CH_2)_n-NR^5$(COalk), $-(CH_2)_n$-Ar ou $-(CH_2)_n$-Het ou $R^3$ et $R^4$ formant ensemble, avec l'atome d'azote auquel ils sont liés, un hétérocycle qui peut aussi être mono ou polysubstitué par un groupe alkyle en $C_1$-$C_6$, cycloalkyle en $C_3$-$C_7$, alcoxy en $C_1$-$C_4$ amino, alkylamino en $C_1$-$C_4$, di(alkyl en $C_1$-$C_4$)amino, hydroxy, acétoxy, benzyle, phénéthyle ou Ar;

$R^5$ et $R^6$ représentant indépendamment l'un de l'autre un atome d'hydrogène, un groupe alkyle en $C_1$-$C_6$, alcényle en $C_3$-$C_6$, cycloalkyle en $C_3$-$C_7$, benzyle, phénéthyle ou Ar; $R^7$ représentant un groupe alkyle en $C_1$-$C_6$, cycloalkyle en $C_3$-$C_7$, benzyle, phényle ou phényle mono ou polysubstitué par un groupe alkyle en $C_1$-$C_4$,

un atome de fluor, chlore ou un groupe nitro;
alk représentant un groupe alkyle en $C_1$-$C_6$;
Ar représentant un groupe aryle avec de 6 à 12 atomes de carbone, qui peut également être mono ou polysubstitué par un groupe alkyle en $C_1$-$C_4$, alcoxy en $C_1$-$C_4$, amino, alkylamino en $C_1$-$C_4$, di(alkyl en $C_1$-$C_4$) amino, alcanoylamino en $C_1$-$C_6$, sulfamoyle, un atome de fluor, de chlore, de brome, un groupe hydroxy, acétoxy, nitro, trifluorométhyle ou cyano;
Het représentant un groupe hétérocyclique avec de 1 à 3 hétéroatomes, qui peut être également mono ou polysubstitué par un groupe alkyle en $C_1$-$C_4$, alcoxy en $C_1$-$C_4$, amino, alkylamino en $C_1$-$C_4$, di(alkyl en $C_1$-$C_4$) amino, alcanoylamino en $C_1$-$C_6$, un atome de fluor, de chlore, de brome, un groupe hydroxy, acétoxy, nitro, cyano ou Ar;
n vaut 0, 1, 2, 3 ou 4,
m vaut 1, 2, 3 ou 4,
p vaut 1, 2 ou 3;

ainsi que leurs sels pharmacologiquement acceptables.

**2.** Dérivés de l'acide hydroxyméthylfurazannecarboxylique de formule générale I selon la revendication 1, caractérisés en ce que, X représente $NR^3R^4$ et en ce qu'un des groupes $R^3$ et $R^4$ représente un atome d'hydrogène et en ce que l'autre groupe correspond à l'une des définitions données dans la revendication 1, ou en ce que $R^3$ et $R^4$ forment ensemble, avec l'atome d'azote auquel ils sont liés, un hétérocycle qui peut aussi être mono ou polysubstitué par un groupe alkyle en $C_1$-$C_6$, cycloalkyle en $C_3$-$C_7$, alcoxy en $C_1$-$C_4$, amino, alkyl en $C_1$-$C_4$-amino, di (alkyl en $C_1$-$C_4$)amino, hydroxy, acétoxy, benzyle, phénéthyle ou Ar

**3.** Dérivés de l'acide hydroxyméthylfurazannecarboxylique de formule générale I selon la revendication 1 et/ou 2, caractérisés en ce que, X représente $NR^3R^4$ et en ce qu'un des groupes $R^3$ et $R^4$ représente un atome d'hydrogène et l'autre un atome d'hydrogène, un groupe alkyle en $C_1$-$C_6$, $-(CH_2)_nNR^5R^6$, n étant 1, 2, 3 ou 4 et $R^5$ et $R^6$ représentant un groupe alkyle en $C_1$-$C_6$, un groupe $-(CH_2)_nOR^5$, n étant 2, 3 ou 4 et $R^5$ représentant un atome d'hydrogène ou un groupe alkyle en $C_1$-$C_4$, $-(CH_2)_nAr$, n étant 0, 1, 2, 3 ou 4 et Ar représentant un groupe phényle non substitué ou mono ou polysubstitué, ou $-(CH_2)_nHet$, n étant 1, 2 ou 3 ou 4 et Het représentant un radical hétérocyclique, qui contient comme hétéroatome 1 ou 2 atomes d'azote ou 1 atome d'azote et 1 atome d'oxygène et qui peut être mono ou polysubstitué par des groupes méthyle, ou en ce que $R^3$ et $R^4$ forment ensemble, avec l'atome d'azote auquel ils sont liés, un hétérocycle qui peut aussi être mono ou polysubstitué par des groupes méthyle.

**4.** Dérivés de l'acide hydroxyméthylfurazannecarboxylique de formule générale I selon une ou plusieurs des revendications 1 à 3, caractérisés en ce que, X représente $NR^3R^4$ et en ce qu'un des groupes $R^3$ et $R^4$ représente un atome d'hydrogène et en ce que l'autre représente un atome d'hydrogène, un groupe alkyle en $C_1$-$C_6$, $-(CH_2)_nNR^5R^6$, n étant 1, 2, 3 et $R^5$ et $R^6$ représentant un groupe alkyle en $C_1$-$C_6$, $-(CH_2)_nAr$ n étant 1 ou 2 et Ar représentant un groupe phényle non substitué ou mono ou bisubstitué, ou $-(CH_2)_nHet$, n étant 1, 2 ou 3 et Het représentant un radical pyridyle ou imidazolyle, ou en ce que les groupes $R^3$ et $R^4$ forment ensemble, avec l'atome d'azote auquel ils sont liés, un noyau pyrrolidine ou un noyau pipérazine qui est substitué par un groupe méthyle.

**5.** Dérivés de l'acide hydroxyméthylfurazannecarboxylique de formule générale I selon la revendication 1, caractérisés en ce que l'un des groupes $R^1$ et $R^2$ représente un groupe hydroxyméthyle et l'autre un groupe

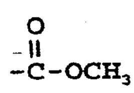 , 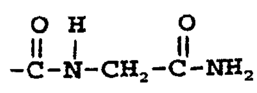

ou de préférence

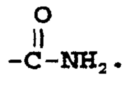

**6.** Dérivés de l'acide hydroxyméthylfurazannecarboxylique de formule générale I selon une ou plusieurs des revendications 1 à 5, caractérisés en ce que $R^1$ représente le groupe hydroxyméthyle.

**7.** Dérivés de l'acide hydroxyméthylfurazannecarboxylique de formule générale I selon une ou plusieurs des revendications 1 à 5, caractérisés en ce que $R^2$ représente le groupe hydroxyméthyle.

**8.** 4-Hydroxyméthyl-2-oxyfurazanne-3-carboxamide

**9.** Procédé de préparation de dérivés de l'acide hydrométhylfurazanne-carxylique de formule générale I selon une ou plusieurs des revendications 1 à 8, caractérisé en ce que l'on oxyde le composé de formule II pour obtenir le composé de formule III

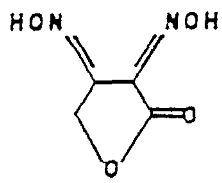

(II)

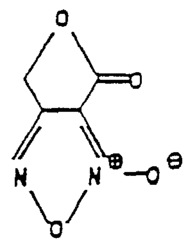

(III)

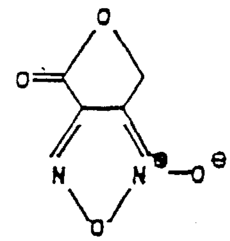

(IV)

ou de formule IV ou un mélange des composés des formules III et IV et en faisant réagir ensuite les composés de formule III et/ou IV avec une amine $R^3R^4NH$, $R^3$ et $R^4$ étant comme définis dans les revendications 1 à 8, pour obtenir les composés de formule générale I, dans laquelle un des groupes $R^1$ et $R^2$ représente $-CONR^3R^4$,
ou l'on fait réagir les composés des formules III et/ou IV avec un alcool $R^7OH$, $R^7$ étant défini comme indiqué dans la revendication 1, pour obtenir les composés des formules générales Ic ou Id

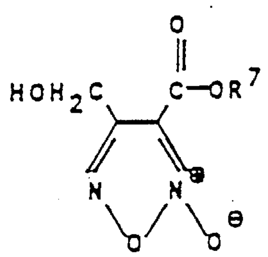

(Ic)

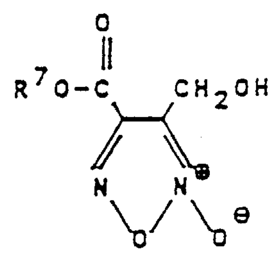

(Id)

ou un mélange des composés de formules générales Ic et Id et on transforme éventuellement les composés de formules générales Ic et/ou Id avec une amine $R^3R^4NH$ en composés de formule générale I dans laquelle l'un des groupes $R^1$ et $R^2$ représente $-CONR^3R^4$.
ou en ce que l'on fait réagir le composé de formule II avec une amine $R^3R^4NH$, $R^3$ et $R^4$ étant comme définis dans les revendications 1 à 8, pour obtenir un composé de formule générale V

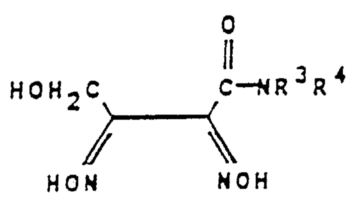

(V)

et on oxyde ce dernier en composés de formule générale I dans laquelle l'un des groupes $R^1$ ou $R^2$ représente $CONR^3R^4$.
et en ce qu'on sépare éventuellement le mélange des composés isomères de formule générale I ou le mélange des isomères des composés des formules générales III et IV ou des formules générales Ic et Id ou des sels de ces composés selon des procédés connus en soi,
et en ce que l'on transforme un composé de formule générale I ou un de ses sels en un sel pharmacologiquement acceptable.

**10.** Dérivés de l'acide hydroxyméthylfurazanne-carboxylique de formule générale I selon une ou plusieurs des revendications 1 à 8 et de leurs sels pharmacologiquement acceptables pour l'administration comme médicament.

**11.** Dérivés de l'acide hydroxyméthylfurazanne-carboxylique de formule générale I selon une ou plusieurs des revendications 1 à 8 et de leurs sels pharmacologiquement acceptables pour la lutte contre et la prophylaxie des maladies du système cardio-vasculaire, en particulier de l'angine de poitrine, ou pour le traitement des dysfonctions érectiles.

**12.** Préparation pharmaceutique, caractérisée en ce qu'elle contient, comme principe actif, un ou plusieurs des dérivés de l'acide hydroxyméthylfurazanne-carboxylique de formule générale I selon une ou plusieurs des revendications 1 à 8 et/ou un ou plusieurs de leurs sels pharmacologiquement acceptables avec des véhicules et des additifs pharmacologiquement acceptables et éventuellement encore un ou plusieurs autres principes pharmacologiques actifs.